(19) Europäisches Patentamt
European Patent Office
Office européen des brevets

(11) Veröffentlichungsnummer: **0 078 764**
**B1**

(12) # EUROPÄISCHE PATENTSCHRIFT

(45) Veröffentlichungstag der Patentschrift: **02.01.86**

(51) Int. Cl.⁴: **C 12 Q 1/56**, C 12 Q 1/36

(21) Anmeldenummer: **82810427.3**

(22) Anmeldetag: **13.10.82**

(54) **Verfahren zur quantitativen Bestimmung von Blutgerinnungsfaktor XII in Humanplasma.**

(30) Priorität: **02.11.81 CH 6972/81**

(43) Veröffentlichungstag der Anmeldung: **11.05.83 Patentblatt 83/19**

(45) Bekanntmachung des Hinweises auf die Patenterteilung: **02.01.86 Patentblatt 86/01**

(84) Benannte Vertragsstaaten: **AT BE CH DE FR GB IT LI NL SE**

(56) Entgegenhaltungen: **US-A- 4 275 153**

(73) Patentinhaber: **Pentapharm A.G.**
**Engelgasse 109**
**CH-4052 Basel (CH)**

(72) Erfinder: **Svendsen, Lars Gundro**
**Reichensteinerstrasse 15**
**CH-4153 Reinach/BL (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 078 764 B1

**Beschreibung**

Die vorliegende Erfindung betrifft ein Verfahren zur quantitativen Bestimmung von Gerinnungsfaktor XII (Hageman-Faktor) in Humanplasma durch Aktivierung des im Plasma enthaltenen Faktors XII (Proenzym) zu Faktor XIIa (Enzym) und direkte Umsetzung des letzteren mit einem enzymatisch spaltbaren Substrat.

Der Faktor XII ist der erste Faktor, der in der endogenen Blutgerinnungskaskade aktiviert wird. Da dieser Faktor eine Schlüsselrolle nicht nur im Gerinnungssystem, sondern auch im Kallikrein-Kinin-System und im fibrinolytischen System spielt, ist es wichtig, diesen Faktor im Blutplasma quantitativ bestimmen zu können. Die heute am meisten verwendete Bestimmungsmethode besteht darin, dass man eine Plasmaprobe mit Kaolin inkubiert, um den Hageman-Faktor maximal zu aktivieren, das Aktivierungsgemisch mit Hageman-Faktor-Mangelplasma inkubiert, nach einer bestimmten Zeit das Inkubationsgemisch durch Zugabe von Calciumionen recalzifiziert und die Gerinnungszeit des recalzifizierten Gemisches misst. Die Gerinnungszeit ist annähernd eine Funktion der Konzentration des Hageman-Faktors in der Testprobe (siehe z. B. Oscar D. Ratnoff in « Thrombosis and Bleeding Disorders », 1971, Georg Thieme Verlag, Stuttgart, Seiten 215-218). Der Nachteil dieser Bestimmungsmethode besteht darin, dass man eine Grösse misst, die das Resultat einer langen Reaktionskaskade und daher von einer grossen Zahl von Reaktionsparametern abhängig ist. Erschwerend kommt dazu, dass man schwer zugängliches Hageman-Faktor-Mangelplasma zur Erstellung von Eichkurven benötigt.

In der USA-Patentschrift Nr. 4.275.153 wird vorgeschlagen, die Verbindung H-D-Phe-Pro-Arg-AIE (AIE = 5-Aminoisophthalsäure-dimethylester) als Substrat für die Bestimmung von Faktor XIIa in Blutplasma zu verwenden. Faktor XIIa spaltet aus dem Substrat enzymatisch eine fluoreszierende Verbindung ab, deren Menge fluoreszenzphotospektrometrisch gemessen werden kann. Aus der ermittelten Menge kann die im Blutplasma ursprünglich vorhandene Menge des Faktors XIIa bestimmt werden. Die mit dieser Methode erzielten Resultate sind jedoch unbrauchbar, da die Verbindung H-D-Phe-Pro-Arg-AIE nicht nur durch Faktor XIIa, sondern auch durch das ebenfalls im Blutplasma enthaltene Kallikrein gespalten wird, und zwar etwa fünfmal stärker als durch Faktor XIIa.

Es wurde nun gefunden, dass man die enzymatische Aktivität des aus Faktor XII gebildeten Faktors XIIa in Blutplasma direkt und quantitativ messen kann, indem man gewisse chromogene Tripeptidderivate als spaltbare Substrate verwendet.

Das Verfahren gemäss der Erfindung ist dadurch gekennzeichnet, dass man das Plasma mit einem Aktivator behandelt, um den im Plasma enthaltenen Faktor XII in Faktor XIIa umzuwandeln, den letzteren mit einem Tripeptidderivat der folgenden allgemeinen Formel :

$$\text{H-D-NH-}\underset{\substack{|\\R^2}}{\text{CH}}\text{-CO-Gly-L-Arg-}R^1$$

in welcher $R^1$ eine enzymatisch abspaltbare chromogene oder fluorogene substituierte Aminogruppe und $R^2$ einen geradkettigen oder verzweigten Alkylrest mit 1 bis 4 Kohlenstoffatomen oder einen Benzyl-, p-Hydroxybenzyl-, Cyclohexylmethyl- oder 4-Hydroxycyclohexylmethyl-Rest darstellen, oder einem Salz davon mit einer organischen Säure oder Mineralsäure zur Reaktion bringt und die Menge des durch Faktor XIIa enzymatisch aus dem Tripeptidderivat freigesetzten farbigen oder fluoreszierenden Spaltproduktes $R^1H$ photometrisch, spektrophotometrisch oder fluoreszenzspektrophotometrisch misst.

Als Aktivator kann man beispielsweise ein Kephalin und Ellagsäure enthaltendes Präparat oder Kaolin verwenden. Die Aktivierung des Faktors XII wird zweckmässigerweise bei etwa 0 °C durchgeführt. Die Umsetzung des Faktors XIIa mit dem Tripeptidderivat wird vorzugsweise in Gegenwart von Sojabohnentrypsininhibitor in einem Puffersystem bei einem pH von 7,4 bis 8,0, vorzugsweise 7,7, und bei einer Ionenstärke von 0,025 bis 0,2, vorzugsweise 0,05, durchgeführt.

Die Salze der Tripeptidderivate können Salze mit Mineralsäuren, z. B. HCl, HBr, $H_2SO_4$ oder $H_3PO_4$, oder einer organischen Säure, z. B. Ameisen-, Essig-, Propion-, Milch-, Zitronen-, Oxal-, Wein-, Benzoe-, Phthal-, Trichloressig- oder Trifluoressigsäure, sein.

Die gemäss der Erfindung verwendeten Tripeptidderivate können nach an sich bekannten Methoden hergestellt werden.

Unter die obige allgemeine Formel fallen beispielsweise die folgenden einzelnen Tripeptidderivate : 2AcOH · H-D-Ala-Gly-Arg-pNA, 2AcOH · H-D-But-Gly-Arg-pNA, 2AcOH · H-D-Val-Gly-Arg-pNA, 2AcOH · H-D-Nval-Gly-Arg-pNA, 2AcOH · H-D-Leu-Gly-Arg-pNA, 2AcOH · H-D-Nleu-Gly-Arg-pNA, 2AcOH · H-D-Ile-Gly-Arg-pNA, 2AcOH · H-D-Phe-Gly-Arg-pNA, 2AcOH · H-D-Tyr-Gly-Arg-pNA, 2AcOH · H-D-CHA-Gly-Arg-pNA, 2AcOH · H-D-CHT-Gly-Arg-pNA.

Die in der obigen Liste verwendeten Abkürzungen haben die folgende Bedeutung :

AcOH = Essigsäure
Ala = Alanyl
Arg = Arginyl

But = α-Aminobutyryl
CHA = 3-Cyclohexyl-alanyl
CHT = 3-(4-Hydroxycyclohexyl)-alanyl
Gly = Glycyl
Ile = Isoleucyl
Leu = Leucyl
Nleu = Norleucyl
Nval = Norvalyl
Phe = Phenylalanyl
Tyr = Tyrosyl
Val = Valyl

Die beiden Aminosäuregruppen, die an die D-Aminosäuregruppe angeknüpft sind, weisen die L-Form auf.

Die oben genannten Tripeptidderivate sind an sich bekannte Verbindungen, in welchen die p-Nitrophenylaminogruppe durch eine andere chromogene oder fluorogene substituierte Aminogruppe, z. B. durch eine 1-Carboxy-2-nitro-phenyl-(5)-amino-, 1-Sulfo-2-nitro-phenyl-(5)-amino, β-Naphthylamino-, 4-Methoxy-β-naphthylamino-, 5-Nitro-α-naphthylamino-, Chinonyl-(5)-amino-, 8-Nitrochinonyl-(5)-amino-, 4-Methyl-cumaryl-(7)-amino- oder 1,3-Di(methoxycarbonyl)-phenyl-(5)-amino-gruppe (abgeleitet von 5-Amino-isophthalsäure-dimethylester) ersetzt sein kann.

Zur Durchführung des erfindungsgemässen Verfahrens kann man wie folgt vorgehen.

Man mischt das Citratplasma, dessen Gehalt an Faktor XII bestimmt werden soll, mit einem wässrigen, Kephalin und Ellagsäure enthaltenden Präparat, z. B. Actin® (Firma DADE, Miami, USA) oder Cephotest® (Firma Nyegaard & Co. As, Oslo, Norwegen) und inkubiert die Mischung während einiger Minuten, vorzugsweise 4 Minuten, bei tiefer Temperatur, vorzugsweise 0 °C, um den Faktor XII in Faktor XIIa überzuführen. Das Arbeiten bei tiefen Temperaturen ist wichtig, um die Bildung von Enzymkomplexen mit dem Plasmainhibitor $\alpha_2$-Makroglobulin zu unterdrücken, weil diese Komplexe die als Substrate verwendeten Tripeptidderivate enzymatisch spalten könnten, allerdings mit kleineren Geschwindigkeiten als die entsprechenden freien Enzyme. Ein aliquoter Teil des erhaltenen Aktivierungsgemisches wird dann einem Testsystem zugesetzt, welches durch Mischen eines Puffers, z. B. Trisimidazolpuffer, der ein pH von 7,4 bis 8,0, vorzugsweise 7,7, und eine Ionenstärke von 0,025 bis 0,2, vorzugsweise 0,05, aufweist und Sojabohnentrypsininhibitor enthält, mit einer wässrigen Lösung eines der oben definierten Tripeptidderivate und einer wässrigen Lösung des Aktivators (Actin oder Cephotest) und Präinkubation während einiger Minuten bei der Temperatur, bei welcher die Spaltung des Tripeptidderivates gemessen werden soll, hergestellt worden ist. Man misst nun die pro Zeiteinheit freigesetzte Menge des farbigen oder fluoreszierenden Spaltproduktes durch photometrische, spektrophotometrische oder fluoreszenzspektrophotometrische Methoden. Wenn das Spaltprodukt p-Nitroanilin ist, wird die Messung vorzugsweise bei 405 nm durchgeführt.

Der im Testsystem enthaltene Sojabohnentrypsininhibitor hat die Funktion, das bei der Aktivierung von Faktor XII zu Faktor XIIa gleichzeitig aktivierte Plasmakallikrein selektiv zu hemmen, um dessen enzymatische Wirkung auf das als Substrat verwendete Tripeptidderivat zu unterbinden. Andererseits übt Sojabohnentrypsininhibitor auf Faktor XIIa keine hemmende Wirkung aus.

Die erfindungsgemässe Bestimmungsmethode bringt gegenüber der bekannten älteren Bestimmungsmethode mehrere Vorteile mit sich, insofern als sie eine direkte Bestimmung des Faktors XII über den aktivierten Faktor XIIa ermöglicht, dessen enzymatische Aktivität mittels der oben definierten Tripeptidderivate in einer einzigen Stufe ermittelt werden kann. Bei der älteren Methode wurde die Aktivität des Faktors XIIa indirekt mittels der Gerinnungszeit gemessen. Die Gerinnung findet erst statt, nachdem die durch Faktor XIIa ausgelöste vollständige endogene Gerinnungskaskade durchlaufen ist. Um vergleichbare Messwerte zu erhalten, ist man gezwungen, Hageman-Faktor-Mangelplasma zu verwenden, welches dazu dient, den Einfluss des Faktors XIIa auf die Gerinnungszeit zu ermitteln und zu diesem Zweck Eichkurven aufzustellen. Die ältere Methode, bei welcher das Endmessresultat erst nach Ablauf komplizierter ineinandergreifender enzymatischer Prozesse erhalten wird, ist mit zahlreichen unkontrollierbaren Fehlerquellen behaftet und ist demgemäss ungenau. Es ist äusserst schwierig, ein Mangelplasma zu erhalten, das völlig frei von Faktor XII ist. Die heutige Tendenz in klinischen Laboratorien geht dahin, automatische Analysengeräte zu verwenden, mit welchen Analysen schnell und genau mit einem Minimum an personellem Aufwand durchgeführt werden können. Die erfindungsgemässe Bestimmungsmethode eignet sich in hervorragender Weise für solche automatischen Analysengeräte, während die ältere Methode nur durch geschultes Laborpersonal manuell in zeitraubender Weise semiquantitativ durchgeführt werden kann.

Das erfindungsgemässe Verfahren kann in der im nachfolgenden Beispiel beschriebenen Weise durchgeführt werden.

## Beispiel

Ein Testsystem, bestehend aus 0,75 ml Trisimidazol-Puffer mit pH 7,7 und Ionenstärke 0,05, welcher

0,1 mg Sojabohnentrypsininhibitor pro ml enthält, 0,25 ml $2 \times 10^{-3}$ molarer wässriger Lösung von 2AcOH · H-D-Leu-Gly-Arg-pNA und 0,20 ml wässrigem aktiviertem Cephaloplastinreagens (Actin® der Firma DADE, Miami, USA), wird während 4 Minuten bei 37 °C präinkubiert. Dem Präinkubat wird 0,05 ml eines Inkubats zugesetzt, welches durch Mischen von 0,1 ml normalem Citratplasma und 0,2 ml wässrigem aktiviertem Cephaloplastinreagens und Inkubation während 4 Minuten bei 0 °C erhalten worden ist. Nach dem Mischen der beiden Komponenten wird die Menge des durch Faktor XIIa freigesetzten p-Nitroanilins bei 405 nm spektrophotometrisch während 5 Minuten kontinuierlich gemessen. Aus der Zunahme der optischen Dichte pro Minute wird die Aktivität des Faktors XIIa in Enzymeinheiten pro ml Plasma berechnet.

Nach dieser Methode werden in Normalplasma durchschnittlich 0,15 bis 0,30 Enzymeinheiten Faktor XIIa pro ml bestimmt. Da 1 Molekül Faktor XII bei der Aktivierung 1 Molekül Faktor XIIa liefert, sind die für Faktor XIIa ermittelten Werte gleichzeitig ein Mass für die ursprünglich im Plasma vorhandene Menge an Faktor XII.

## Patentansprüche

1. Verfahren zur quantitativen Bestimmung des Blutgerinnungsfaktors XII in Humanplasma, dadurch gekennzeichnet, dass man das Plasma mit einem Aktivator behandelt, um den im Plasma enthaltenen Faktor XII in Faktor XIIa umzuwandeln, den letzteren mit einem Tripeptidderivat der Formel

```
H-D-NH-CH-CO-Gly-L-Arg-R1
       |
       R2
```

in welcher $R^1$ eine enzymatisch abspaltbare chromogene oder fluorogene substituierte Aminogruppe und $R^2$ einen geradkettigen oder verzweigten Alkylrest mit 1 bis 4 Kohlenstoffatomen oder einen Benzyl-, p-Hydroxybenzyl-, Cyclohexylmethyl- oder 4-Hydroxycyclohexylmethyl-Rest darstellen, oder einem Salz davon mit einer organischen Säure oder Mineralsäure zur Reaktion bringt und die Menge des durch Faktor XIIa enzymatisch aus dem Tripeptidderivat freigesetzten farbigen oder fluoreszierenden Spaltproduktes $R^1H$ photometrisch, spektrophotometrisch oder fluoreszenzspektrophotometrisch misst.

2. Verfahren nach Patentanspruch 1, dadurch gekennzeichnet, dass man als Aktivator ein Kephalin und Ellagsäure enthaltendes Präparat oder Kaolin verwendet.

3. Verfahren nach Patentanspruch 1 und 2, dadurch gekennzeichnet, dass die Umsetzung des Faktors XIIa mit dem Tripeptidderivat in Gegenwart von Sojabohnentrypsininhibitor durchgeführt wird.

4. Verfahren nach Patentanspruch 1 bis 3, dadurch gekennzeichnet, dass die Umsetzung in einem Puffersystem bei pH von 7,4 bis 8,0 und bei einer Ionenstärke von 0,025 bis 0,2 durchgeführt wird.

5. Verfahren nach Patentanspruch 1, dadurch gekennzeichnet, dass die Aktivierung des Faktors XII bei 0 °C durchgeführt wird.

6. Verfahren nach Patentanspruch 1, dadurch gekennzeichnet, dass $R^1$ eine p-Nitrophenylamino-, 1-Carboxy-2-nitro-phenyl-(5)-amino-, 1-Sulfo-2-nitro-phenyl-(5)-amino-, β-Naphthylamino-, 4-Methoxy-β-naphthylamino-, 5-Nitro-α-naphthylamino, Chinonyl-(5)-amino, 8-Nitro-chinonyl-(5)-amino-, 4-Methyl-cumaryl-(7)-amino- oder 1,3-Di(methoxycarbonyl)-phenyl-(5)-amino-gruppe (abgeleitet von 5-Amino-isophthalsäure-dimethylester) darstellt.

## Claims

1. Method for quantitatively assaying blood coagulation factor XII in human plasma, characterized by treating plasma with an activator to convert factor XII present in the plasma into factor XIIa, reacting the latter with a tripeptide derivative of formula

```
H-D-NH-CH-CO-Gly-L-Arg-R1
       |
       R2
```

wherein $R^1$ represents a chromogenic or fluorogenic substituted amino group capable of being split off enzymatically, $R^2$ represents a straight chain or branched alkyl radical having 1 to 4 carbon atoms or a benzyl, p-hydroxybenzyl, cyclohexylmethyl or 4-hydroxycyclohexylmethyl radical, or a salt thereof with an organic acid or a mineral acid, and measuring the quantity of coloured or fluorescent split product $R^1H$ released enzymatically by factor XIIa from the tripeptide derivative by photometric, spectrophotometric or fluorescence-spectrophotometric methods.

2. Method according to claim 1, characterized by using a preparation containing cephalin and ellagic acid, or kaolin, as the activator.

3. Method according to claims 1 and 2, characterized by carrying out the reaction of factor XIIa with the tripeptide derivative in the presence of soybean trypsin inhibitor.

4. Method according to claims 1 to 3, characterized by carrying out the reaction in a buffer system at pH 7.4 to 8.0 and an ionic strength of 0.025 to 0.2.

5. Method according to claim 1, characterized by carrying out the activation of factor XII at 0 °C.

6. Method according to claim 1, characterized by the fact that $R^1$ represents a p-nitrophenylamino, 1-carboxy-2-nitrophenyl-(5)-amino, 1-sulfo-2-nitro-phenyl-(5)-amino, β-naphthylamino, 4-methoxy-β-naphthylamino, 5-nitro-α-naphthylamino, quinonyl-(5)-amino, 8-nitro-quinonyl-(5)-amino, 4-methylcoumaryl-(7)-amino or 1,3-di(methoxycarbonyl)-phenyl-(5)-amino group (derived from dimethyl 5-aminoisophthalate).

**Revendications**

1. Procédé pour le dosage quantitatif du facteur de coagulation sanguine XII dans le plasma humain, caractérisé en ce que l'on traite le plasma avec un activateur pour transformer le facteur XII contenu dans le plasma en facteur XIIa, qu'on fait réagir ce dernier avec un dérivé tripeptidique de formule

$$\begin{array}{l} \text{H-D-NH-}\underset{\displaystyle R^2}{\underset{|}{\text{CH}}}\text{-CO-Gly-L-Arg-}R^1 \end{array}$$

dans laquelle $R^1$ représente un groupe amino substitué, chromogène ou fluorogène, dissociable par voie enzymatique, et $R^2$ représente un radical alkyl linéaire ou ramifié possédant 1 à 4 atomes de carbone ou un radical benzyl, p-hydroxybenzyl, cyclohexylméthyl ou 4-hydroxycyclohexylméthyl, ou avec un de ses sels avec un acide organique ou minéral, et qu'on mesure, par photométrie, spectrophotométrie ou spectrophotométrie de fluorescence, la quantité de produit de scission $R^1H$ coloré ou fluorescent formé par l'action enzymatique du facteur XIIa sur le dérivé tripeptidique.

2. Procédé selon la revendication 1, caractérisé par l'utilisation d'une préparation contenant une céphaline et de l'acide ellagique, ou de kaolin, comme activateur.

3. Procédé selon les revendications 1 et 2, caractérisé en ce que la réaction du facteur XIIa avec le dérivé tripeptidique est effectuée en présence d'inhibiteur de trypsine de soja.

4. Procédé selon les revendications 1 à 3, caractérisé en ce que la réaction est effectuée dans un système tampon à un pH de 7,4 à 8,0 et une intensité ionique de 0,025 à 0,2.

5. Procédé selon la revendication 1, caractérisé en ce que l'activation du facteur XII est effectuée à 0 °C.

6. Procédé selon la revendication 1, caractérisé en ce que $R^1$ représente un groupe p-nitrophénylamino, 1-carboxy-2-nitro-phényl-(5)-amino, 1-sulfo-2-nitro-phényl-(5)-amino, β-naphtylamino, 4-méthoxy-β-naphtylamino, 5-nitro-α-naphtylamino, quinonyl-(5)-amino, 8-nitro-quinonyl-(5)-amino, 4-méthyl-coumaryl-(7)-amino ou 1,3-di(méthoxycarbonyl)-phényl-(5)-amino (dérivé du 5-amino-isophtalate diméthylique).